(19) Europäisches Patentamt
European Patent Office
Office européen des brevets

(11) EP 1 439 166 A1

(12) **DEMANDE DE BREVET EUROPEEN**

(43) Date de publication:
**21.07.2004 Bulletin 2004/30**

(51) Int Cl.[7]: **C07C 227/18**, C07C 229/24

(21) Numéro de dépôt: **03293080.2**

(22) Date de dépôt: **10.12.2003**

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **20.12.2002 FR 0216344**

(71) Demandeur: **ISOCHEM**
**75004 Paris (FR)**

(72) Inventeurs:
- **Vitrant, Anne Marie**
  **91760 Itteville (FR)**
- **Ferruccio, Laurence**
  **91810 Vert Le Grand (FR)**
- **Vincent, Charles-Henry**
  **60460 Precy sur Oise (FR)**

(74) Mandataire: **Waligorski, Carol**
**SNPE - Service Propriété Industrielle**
**12, Quai Henri IV**
**75004 Paris (FR)**

(54) **Procédé de préparation d'esters oméga-benzyliques d'amino-diacides et d'alcanesulfonates de ces esters ainsi que ces alcanesulfonates**

(57) L'invention concerne un procédé de préparation d'un ester ω-benzylique d'un amino-diacide, caractérisé en ce que l'on fait réagir l'amino-diacide avec un dérivé de l'alcool benzylique de formule (I)

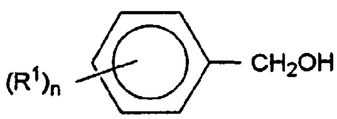

dans laquelle le ou les substituants $R^1$, identiques ou différents, représentent un atome d'hydrogène, un groupe alkyle en $C_1$ à $C_4$, un groupe alcoxy en $C_1$ à $C_4$, ou un atome d'halogène, et n est égal à 1, 2 ou 3,
en présence d'au moins une mole par mole de l'amino-diacide d'un acide alcanesulfonique, éventuellement en présence d'un solvant.

On obtient grâce à ce procédé les alcanesulfonates des esters ω-benzyliques d'amino-diacides intermédiaires et les esters ω-benzyliques d'amino-diacides avec un bon rendement et une excellente pureté.

EP 1 439 166 A1

## Description

L'invention concerne un procédé de préparation d'esters ω-benzyliques d'amino-diacides carboxyliques, un procédé de préparation des alcanesulfonates de ces esters ainsi que les alcanesulfonates de ces esters eux-mêmes.

Les esters ω-benzyliques d'amino-diacides et leurs sulfonates, en particulier ceux des acides aspartique et glutamique ou de leurs dérivés, sont des composés très utiles pour l'obtention de produits destinés à la médecine et à l'agriculture.

Des procédés de préparation de ces composés ont été décrits. Ils consistent notamment à faire réagir l'amino-diacide avec l'alcool benzylique ou un de ses dérivés en présence d'un acide.

Cependant, aucun de ces procédés n'est vraiment satisfaisant. Les rendements sont souvent faibles et les esters contiennent encore de nombreuses impuretés qui les rendent inutilisables sans traitements de purification ultérieurs. Le diester ou l'ester en α se forment dans bien des cas majoritairement. De plus, il se produit fréquemment une racémisation et le dérivé L ou D souhaité n'est pas obtenu.

L'utilisation d'acide sulfurique concentré a été essayée mais ne convient pas. Lorsque l'acide est concentré, la réaction a lieu avec une grande exothermicité qui est incontrôlable. On constate aussi la formation de polymères d'alcool benzylique. Lorsque l'acide est dilué, l'équilibre de la réaction n'est pas favorable à l'estérification. Si l'on remplace l'acide sulfurique par l'acide chlorhydrique, il se forme majoritairement des chlorures benzyliques.

D'autres acides ont été employés tels que les acides benzènesulfonique et paratoluènesulfonique comme mentionné par D.W. CLAYTON et al, dans la revue J. Chem. Soc, 1956, p.374. Généralement la réaction est effectuée à température élevée avec un grand excès d'alcool benzylique ou d'un de ses dérivés. L'excès d'alcool benzylique et les conditions opératoires utilisées entraînent de nombreuses réactions secondaires, en particulier la formation du diester, et conduisent à une racémisation importante.

Il existait, par conséquent, à résoudre le problème de l'obtention des esters ω-benzyliques d'amino-diacides, à l'échelle industrielle, de façon économique, avec un bon rendement, une excellente pureté optique et au moyen d'opérations simples.

Conformément à la présente invention, le problème est résolu en faisant réagir un amino-diacide carboxylique avec un dérivé de l'alcool benzylique de formule (I)

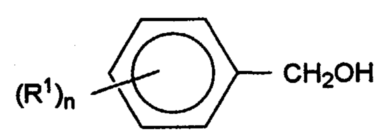

dans laquelle le ou les substituants $R^1$, identiques ou différents, représentent un atome d'hydrogène, un groupe alkyle en $C_1$ à $C_4$, un groupe alcoxy en $C_1$ à $C_4$ ou un atome d'halogène, et n est égal à 1, 2 ou 3,
en présence d'au moins une mole par mole de l'amino-diacide carboxylique d'un acide alcanesulfonique, éventuellement en présence d'un solvant.

Grâce à ce procédé, on obtient les esters ω-benzyliques des amino-diacides et intermédiairement les alcanesulfonates de ces esters, simplement, avec de bons rendements et avec une grande pureté chimique et optique, celles-ci généralement supérieure à 99 %.

Les amino-diacides carboxyliques intéressants comme composés de départ sont notamment les amino-diacides carboxyliques utilisés dans le domaine des peptides. Le radical porteur des deux groupes carboxyliques et du groupe amino peut être très varié et en particulier être un radical aliphatique, cycloaliphatique, arylique, araliphatique, hétérocyclique ou un radical constitué par plusieurs de ces radicaux. Il est substitué ou non, notamment par les substituants habituels des amino-acides. Les substituants sont protégés si nécessaire.

Les amino-diacides peuvent être naturels ou non naturels et se trouvent dans la nature ou sont obtenus par synthèse.

En particulier ce sont les amino-diacides dont les deux fonctions acides carboxylique ne sont pas fixés sur le même atome de carbone ou ne sont pas fixés dans la molécule de façon symétrique par rapport au groupe

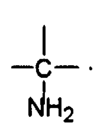

Le groupe amino des amino-diacides peut être protégé par un des groupes protecteurs habituels du groupe

amino tels que ceux utilisés dans la chimie des peptides à condition que celui-ci s'élimine dans les conditions acides de la réaction. Comme groupe protecteur, on peut citer par exemple le groupe tertio-butyloxycarbonyle (BOC).

En particulier, les amino-diacides sont les α-amino-acides carboxyliques naturels ou non porteurs d'un autre groupe carboxyle situé sur un carbone autre que celui en α, sous leurs différentes formes. On peut notamment citer l'acide aspartique, l'acide glutamique et les dérivés de ces acides.

On estérifie les amino-diacides sous leur forme optiquement active D ou L pour obtenir les esters ω-benzyliques D ou L correspondants, pratiquement sans racémisation et avec de bons rendements, l'estérification s'effectuant principalement sur le groupe carboxyle situé en ω.

Les esters ω-benzyliques racémiques peuvent également être obtenus à partir des amino-diacides racémiques correspondants.

Les dérivés de l'alcool benzylique qui sont mis à réagir avec les amino-diacides sont les dérivés de formule (I)

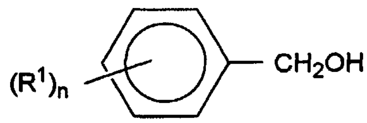

dans laquelle $R^1$ et n ont la signification précédemment indiquée.

Lorsque n est supérieur à 1, les substituants $R^1$ peuvent être identiques ou différents. n est de préférence égal à 1 ou 2. Lorsque $R^1$ représente un atome d'halogène, celui-ci est de préférence le chlore ou le brome.

Comme dérivés de l'alcool benzylique, on peut notamment citer les alcools benzyliques substitués par un ou plusieurs groupes alkyles, tels que l'alcool 4-méthylbenzylique, l'alcool 2,4-diméthylbenzylique, etc., les alcools benzyliques substitués par des groupes alcoxy, tels que l'alcool 4-méthoxybenzylique, etc., les alcools benzyliques substitués par des atomes d'halogène, tel que l'alcool 3-chlorobenzylique, l'alcool 2-bromobenzylique, etc.

De préférence, on effectue l'estérification avec l'alcool benzylique.

La quantité d'alcool benzylique ou de son dérivé utilisée est d'au moins une mole par mole d'amino-diacide et de préférence de 1,2 moles à 3 moles.

Contrairement à l'enseignement de l'art antérieur, on a trouvé qu'il était préférable de réaliser la réaction avec un faible excès d'alcool benzylique ou de son dérivé. On évite ainsi les réactions secondaires et l'apparition de sous-produits indésirables.

Le milieu doit cependant rester agitable. Si nécessaire, on ajoute alors un solvant pour permettre une meilleure agitation du milieu.

Un acide doit être présent dans le milieu pour que l'estérification ait lieu. Les acides utilisés dans les procédés selon l'art antérieur ne donnent pas satisfaction. On a trouvé que de façon surprenante, lorsqu'on utilise un acide alcanesulfonique, le procédé selon l'invention conduit aux esters ω-benzyliques avec de bons rendements et une excellente pureté optique.

Comme acides alcanesulfoniques, on peut citer les acides alcanesulfoniques en $C_1$ à $C_4$, et en particulier l'acide méthanesulfonique, l'acide éthanesulfonique, l'acide propanesulfonique. De préférence, on utilise l'acide méthanesulfonique.

Le schéma réactionnel à partir de l'acide aspartique ou glutamique, de l'alcool benzylique et de l'acide méthanesulfonique est le suivant :

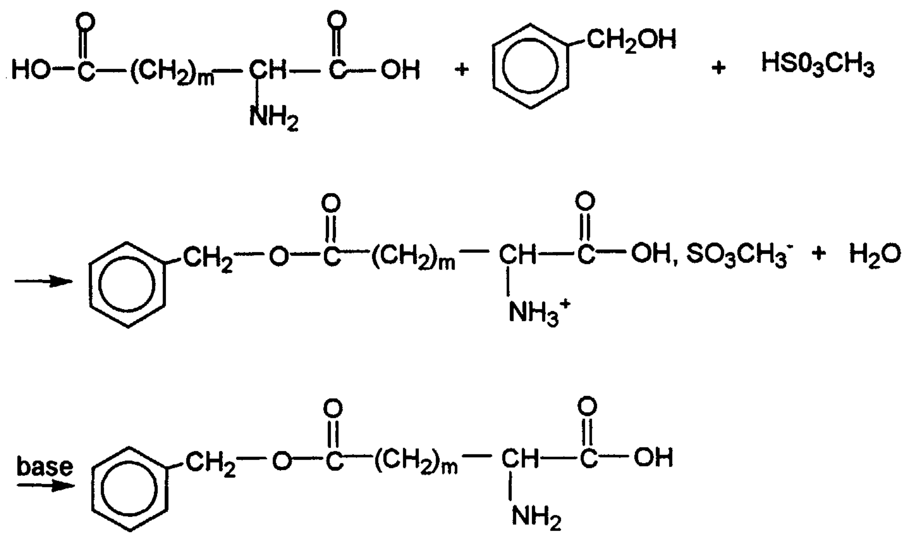


m = 1 ou 2

La quantité nécessaire d'acide alcanesulfonique à ajouter est d'au moins une mole par mole de l'amino-diacide afin de neutraliser la fonction amine. De préférence, on utilise un excès de cet acide sulfonique, notamment une quantité de 1,01 à 2 moles par mole de l'amino-diacide et plus particulièrement de 1,05 à 1,2 mole. Ou bien, on ajoute une mole d'acide alcanesulfonique et on apporte l'excès d'acide, soit de 0,01 à 1 mole, en ajoutant un autre acide tel que par exemple l'acide sulfurique.

Les constituants du mélange réactionnel peuvent être introduits dans un ordre quelconque, en fonction de l'agitabilité du milieu. On peut ainsi mélanger d'abord l'amino-diacide avec l'acide alcanesulfonique en présence d'un solvant puis ajouter dans le milieu le dérivé d'alcool benzylique. Dans certains cas, on préfère mélanger l'amino-diacide, l'alcool benzylique ou son dérivé et éventuellement le solvant, puis ajouter, en particulier progressivement, l'acide alcanesulfonique.

Comme solvants qui peuvent être utilisés, on peut citer les solvants hydrocarbonés aromatiques ou aliphatiques, halogénés ou non, tels que le benzène, le toluène, les xylènes, le chlorobenzène, le cyclohexane, le 1,2-dichloroéthane, le dichlorométhane, le chloroforme.

En particulier conviennent bien, les solvants qui ne solubilisent pas l'alcanesulfonate de l'ester w-benzylique de l'amino-diacide. Ceux qui forment également un bon azéotrope avec l'eau sont aussi intéressants. De préférence, on utilise le toluène.

La quantité en volume (exprimée en litre) de solvant lorsqu'il est présent est généralement comprise entre 0,5 et 10 fois la quantité en poids (exprimée en kg) d'amino-diacide utilisé.

La température de la réaction varie selon le type d'amino-diacides à estérifier mais on a trouvé que l'on améliore nettement les résultats si on choisit de maintenir la température à des valeurs pas trop élevées, en particulier inférieures ou égales à 80°C et plus particulièrement comprises de 30°C à 50°C lorsqu'on utilise l'acide glutamique, et de 30° à 80°C lorsqu'on utilise l'acide aspartique.

Le temps de la réaction quant à lui, varie notamment en fonction de la température et de l'amino-diacide à estérifier. Généralement, il est compris entre 1 heure et 24 heures.

La réaction conduit à la formation de l'ester ω-benzylique de l'amino-diacide sous forme du sel de l'acide alcanesulfonique.

On a de plus trouvé qu'on augmentait encore le rendement du procédé et la pureté de l'ester ω-benzylique si on faisait cristalliser complètement ledit sel alcanesulfonique de l'ester ω-benzylique avant d'effectuer les opérations de récupération de l'ester sous forme libre.

Pour ce faire, lorsqu'un solvant a été utilisé, de préférence on effectue la distillation de l'azéotrope solvant-eau. Cette distillation est réalisée généralement en continu pendant le déroulement de la réaction. Si nécessaire, on l'effectue sous pression réduite afin de maintenir la température du milieu réactionnel dans les limites indiquées précédemment, en particulier à une valeur inférieure ou égale à 80°C.

Eventuellement, on refroidit ensuite le milieu pour que la cristallisation de l'alcanesulfonate de l'ester ω-benzylique soit vraiment complète. L'alcanesulfonate obtenu peut être isolé, par exemple par filtration. Il peut également être rincé avec un solvant organique ne le dissolvant pas, tel que le solvant utilisé dans la réaction, en particulier avec

le toluène pour parfaire l'élimination des impuretés.

Lorsqu'aucun solvant n'est utilisé ou qu'une faible quantité de solvant est présente dans le milieu, on préfère faire cristalliser directement l'alcanesulfonate. Généralement on refroidit le milieu. Eventuellement, on peut utiliser d'autres techniques connues tel qu'un ensemencement. De préférence, on refroidit le milieu très lentement. Plusieurs heures peuvent alors être nécessaires.

L'objet de la présente invention concerne également les sels, alcanesulfonates d'esters ω-benzyliques des amino-diacides carboxyliques. Ce sont des composés nouveaux.

Les différentes parties : restes benzyliques, restes d'amino-diacides et restes d'acides alcanesulfoniques qui constituent ces sels, sont en particulier issues de l'alcool benzylique ou de ses dérivés de formule (I), des amino-diacides et des acides alcanesulfoniques tels que précédemment décrits. Ils peuvent être sous forme optiquement actives telles que D ou L ou sous forme racémique.

En particulier le reste benzylique est issu de l'alcool benzylique, le reste d'amino-diacide carboxylique est issu d'un α-amino-acide carboxylique porteur d'un autre groupe carboxyle fixé sur un carbone autre que celui en α et le reste d'acide alcanesulfonique est issu d'un acide alcanesulfonique en $C_1$ à $C_4$, plus particulièrement de l'acide méthanesulfonique.

On peut les représenter par la formule (II) suivante :

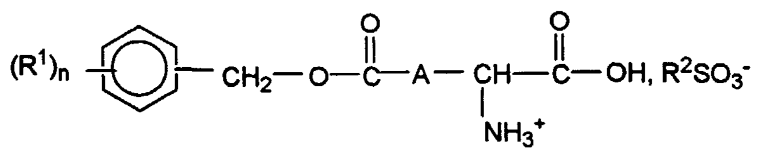

dans laquelle $R^1$ et n sont tels que définis pour la formule (I), A est la partie de la molécule d'un α-amino-acide carboxylique attachée au carbone en α et au groupe carboxyle en ω et $R^2$ représente le reste alcane de l'acide alcanesulfonique.

$R^1$ est de préférence un atome d'hydrogène et $R^2$ est de préférence un reste alcane en $C_1$ à $C_4$, en particulier le radical $CH_3$.

Comme exemples de tels sels, on peut notamment citer le γ-méthanesulfonate glutamate de benzyle et le β-méthanesulfonate aspartate de benzyle, en particulier sous leur forme L ou D.

Ces sels sont obtenus par le procédé de la présente invention tel que précédemment décrit, avec une excellente pureté chimique et optique.

Lorsque l'on veut récupérer l'ester ω-benzylique non plus sous sa forme de sel alcanesulfonique mais sous sa forme libre, on met l'alcanesulfonate de l'ester ω-benzylique, éventuellement après l'avoir dissout dans l'eau, en contact avec une base organique ou minérale, comme par exemple, une solution aqueuse d'ammoniaque, d'un hydroxyde ou d'un carbonate de métal alcalin, tel que de soude ou d'un carbonate de sodium. De préférence, on utilise une solution aqueuse d'ammoniaque.

En particulier, on ajoute la base en quantité suffisante pour amener et maintenir le pH du milieu au point isoélectrique de l'ester à obtenir, soit plus particulièrement à une valeur de 6 à 7 pour l'acide aspartique ou l'acide glutamique.

L'ester libre précipite au fur et à mesure de la montée du pH à la valeur du point isoélectrique. On le récupère alors facilement de façon classique par exemple par filtration.

On a trouvé qu'on améliorait encore la pureté de l'ester libre par élimination d'impuretés en opérant de la façon suivante. Après avoir fait cristalliser l'alcanesulfonate de l'ester ω-benzylique, de préférence lentement, on ne l'isole pas. Mais on le dissout avec de l'eau et on le traite avec une base telle que précédemment indiquée. On ajoute généralement dans le milieu l'eau en quantité suffisante pour le dissoudre. De préférence, on ajoute 2 à 3 volumes (en litre) d'eau par rapport au poids (en kg) de l'amino-diacide engagé ou de l'alcanesulfonate de l'ester ω-benzylique. On élimine éventuellement la phase organique par décantation. Avec l'eau ou après, on ajoute la base organique ou minérale, de préférence une solution aqueuse d'ammoniaque, pour atteindre le pH du point isoélectrique et on opère comme décrit précédemment.

Selon une variante préférée, on facilite les opérations ultérieures de filtration et de lavage de l'ester libre et on élimine de ce fait encore mieux les impuretés en chauffant, après avoir atteint le pH du point isoélectrique, le milieu, de préférence à une température comprise entre 50° et 70°C. On fait ensuite précipiter l'ester, par exemple par refroidissement.

Pour une pureté encore plus élevée, on peut ajouter dans le milieu contenant l'ester à libérer un solvant de l'alcool benzylique ou de son dérivé, par exemple un alcool comme le méthanol, l'éthanol, l'isopropanol, un ester ou une cétone, généralement en quantité comprise entre 1 et 10 volumes (en litre), en particulier entre 3 et 5 volumes,

par rapport au poids (en kg) d'amino-acide utilisé. De préférence, on ajoute ce solvant avec l'eau ou après l'introduction de l'eau, éventuellement avec la base.

Les rendements obtenus en esters ω-benzyliques sont nettement améliorés par rapport à ceux de l'art antérieur. La pureté chimique et optique de ces esters est excellente, supérieure à 99 %.

Les exemples qui suivent illustrent l'invention sans toutefois la limiter.

Exemple 1 : Préparation du γ-glutamate de benzyle.

Dans un réacteur de 5,2 litres équipé, on introduit 1 kg (6,79 mol, 1 éq) d'acide L-glutamique, 1,1 kg (10,17 mol, 1,5 éq) d'alcool benzylique et 1 litre de toluène et on agite le mélange. On introduit ensuite progressivement 0,784 kg (8,15 mol, 1,2 éq) d'acide méthanesulfonique en maintenant la température du mélange à 45°C. On continue à agiter le mélange pendant 2 heures encore à cette température puis on le refroidit à 30°C-32°C et on continue à l'agiter pendant 4 heures à 30°C-32°C.

On ajoute ensuite 2 litres d'eau. Puis on sépare la phase organique et la phase aqueuse par décantation.

La phase aqueuse recueillie est envoyée dans un autre réacteur. Dans cette phase, on ajoute alors 3 litres d'éthanol puis 0,8 litre d'ammoniaque à 22°B pour atteindre un pH de 6,5-7. On chauffe ensuite le mélange à 60°C et on l'agite pendant 2 heures à cette température pour améliorer la forme cristalline du produit.

On le refroidit à une température de 5°C-10°C. Un solide cristallin précipite. On l'essore, on le lave 2 fois avec 1 litre d'éthanol, et 3 fois avec 1 litre d'eau.

On recueille un produit humide que l'on sèche sous vide. On obtient ainsi 1,25 kg (rendement 77%) de γ-L-glutamate de benzyle, dont les caractéristiques sont les suivantes :

$\alpha_D^{20}$ : + 19° (c = 1 dans l'acide acétique)
Point de fusion (Pf) : 167,5°C
Pureté par HPLC : 99,7 %
Diamètre des grains : 95,5 μ (pour 90 % des grains).

De la même façon à partir de l'acide D-glutamique, on a obtenu le γ-D-glutamate de benzyle dont les caractéristiques sont les suivantes :

$\alpha_D^{20}$ : - 19,5° (c = 1, dans l'acide acétique)

Pureté par HPLC : 99 %.

Exemple 2 : Préparation du méthanesulfonate de γ-L-glutamate de benzyle et du γ-L-glutamate de benzyle.

Dans un réacteur de 3 litres équipé et agité, on introduit 1620 ml de toluène, 294 g (2 mol, 1 éq) d'acide L-glutamique et 648 g (6 mol, 3 éq) d'alcool benzylique et on chauffe le mélange à une température de 30°C-35°C.

On ajoute progressivement 240 g (2,5 mol, 1,25 éq) d'acide méthanesulfonique à 99% en maintenant le mélange à la température de 30°C-35°C. On distille l'azéotrope toluène/eau, pendant 4h30, à 30°C-35°C sous une pression de 38 à 47 mbar.

On refroidit le milieu à 15°C. Le méthanesulfonate de γ-L-glutamate de benzyle précipite. On le filtre et on le rince plusieurs fois avec du toluène. On obtient ainsi 653 g de méthanesulfonate de γ-L-glutamate de benzyle humide sous forme d'une poudre cristalline blanche dont les caractéristiques à l'état sec sont les suivantes :

Point de fusion : 123,1°C
RMN$^1$H (200 MHz, DMSO)

Les protons sont repérés par les chiffres 1 à 9 sur la formule ci-dessous :

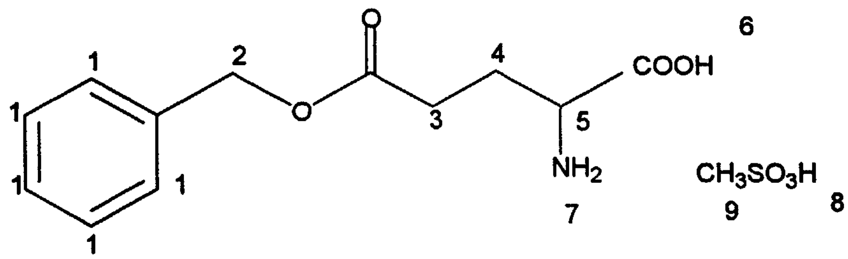

1 : 7,35 ppm, 2 : 5,1 ppm, 3 : 2,6 ppm, 4 : 2,05 pm, 5 : 3,95 ppm, 9 : 2,35 ppm, protons mobiles 8,3 : ppm.

On réintroduit le méthanesulfonate de γ-L-glutamate de benzyle humide obtenu précédemment dans le réacteur qui contient 1,8 litre d'eau et on effectue une nouvelle distillation pour éliminer le reste du toluène. On ajoute ensuite 325 ml d'une solution aqueuse d'ammoniaque à 10% p/p pour que le pH du milieu soit de 6,1 ± 0,2. La température du milieu est de 13°C-15°C. On continue à agiter le mélange pendant 2 heures à ce pH.

On filtre le précipité et on le rince plusieurs fois avec de l'eau. On le sèche à l'étuve à 45°C. On récupère alors 382,8 g (rendement 81 %) de γ-L-glutamate de benzyle sous forme d'une poudre blanche de caractéristiques suivantes :

$\alpha_D^{20}$ : + 20,8° (c = 1 dans l'acide acétique)
pureté déterminée par HPLC : 99,9%
pourcentage d'acide L-glutamique par analyse CCM : < 0,1%
diamètre des grains : 222,7 µ (pour 90 %).

Exemple 3 : Préparation du β-L-aspartate de benzyle

Dans un réacteur de 1 litre équipé, on introduit 100 g (0,75 mol, 1 éq) d'acide L-aspartique, 162 g (1,5 mol, 2 éq) d'alcool benzylique et on agite le mélange. On introduit ensuite progressivement 86,4 g (0,9 mol, 1,2 éq) d'acide méthanesulfonique en laissant monter la température. On amène par chauffage la température à 60°C et on continue à agiter le mélange pendant 12 heures puis on le refroidit à 40°C.

On ajoute ensuite 200 ml d'eau puis 300 ml d'éthanol et ensuite 95 ml d'ammoniaque à 22°B de façon à faire précipiter l'ester à un pH de 6,5-7.

On chauffe ensuite le mélange à 60°C et on l'agite 2 heures à cette température pour améliorer la forme cristalline du produit.

On le refroidit à une température de 5°C-10°C. On essore le précipité cristallin, on le lave 2 fois avec 100 ml d'éthanol et 3 fois avec 100 ml d'eau.

On recueille un produit humide que l'on sèche sous vide. On obtient ainsi 108 g (rendement 64 %) de β-L-aspartate de benzyl dont les caractéristiques sont les suivantes :

aspect : poudre blanche
point de fusion : 212°C
$\alpha_{20}^{D}$ : +27,6° (lu à 1 % dans HCl 1N)
pureté déterminée par HPLC : > 99,9%

En opérant de la même façon à partir de l'acide D-aspartique on obtient le β-D-aspartate de benzyle.

Exemple 4 : Préparation du β-L-aspartate de benzyle

Dans un réacteur de 4 litres équipé, on introduit 532 g (4 mol) d'acide L-aspartique, 665 ml de 1,2-dichloroéthane (DCE), 665 ml de cyclohexane et 318 ml d'acide méthanesulfonique (AMS) 98 % p/p dilué avec 188,5 ml d'eau (soit 4,8 mol d'AMS). Dans ce milieu, on ajoute 827 ml (8 mol) d'alcool benzylique.

On chauffe le milieu vers 76°C et on distille l'azéotrope ternaire eau/DCE/cyclohexane. On observe la précipitation du méthanesulfonate de β-L-aspartate de benzyle quand 135 ml d'eau ont été distillés. La distillation dure 10 heures. La température du milieu avoisine 77°C. La quantité totale d'eau distillée est de 270 ml.

On refroidit le milieu vers 10°C puis on le filtre et on rince le précipité avec 2 fois 530 ml du mélange DCE/cyclohexane (50/50).

Après séchage du précipité, on obtient 861 g de méthanesulfonate de β-L-aspartate de benzyle solide blanc (rendement 66 %). On le solubilise dans 3 volumes d'eau puis on ajuste le pH à 7 ± 0,2 avec de la soude à 30 % p/p. Le précipité est filtré, rincé avec de l'eau puis séché en étuve sous vide (45°C/20mm Hg). Le rendement de cette seconde étape est de 89 % par rapport au méthanesulfonate de l'aspartate de benzyle.

La pureté déterminée par HPLC du β-aspartate de benzyle est de 100 %.

## Revendications

1. Procédé de préparation d'un ester ω-benzylique d'un amino-diacide carboxylique, **caractérisé en ce que** l'on fait réagir l'amino-diacide carboxylique avec un dérivé de l'alcool benzylique de formule (I)

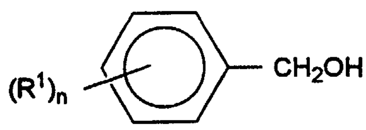

dans laquelle le ou les substituants $R^1$, identiques ou différents, représentent un atome d'hydrogène, un groupe alkyle en $C_1$ à $C_4$, un groupe alcoxy en $C_1$ à $C_4$, ou un atome d'halogène, et n est égal à 1, 2 ou 3, en présence d'au moins une mole par mole de l'amino-diacide carboxylique d'un acide alcanesulfonique, éventuellement en présence d'un solvant.

2. Procédé selon la revendication 1 ou 2, **caractérisé en ce que** l'amino-diacide est un α-amino-acide carboxylique porteur d'un autre groupe carboxyle fixé sur un carbone autre que celui en α.

3. Procédé selon la revendication 2, **caractérisé en ce que** l'amino-diacide est l'acide glutamique ou l'acide aspartique.

4. Procédé selon l'une quelconque des revendications précédentes, **caractérisé en ce que** l'alcool de formule (I) est l'alcool benzylique.

5. Procédé selon l'une quelconque des revendications précédentes, **caractérisé en ce que** la température de la réaction est inférieure ou égale 80°C.

6. Procédé selon l'une quelconque des revendications précédentes, **caractérisé en ce que** l'alcool benzylique ou son dérivé de formule (I) est utilisé en quantité choisie dans la gamme allant de 1,2 à 3 moles par mole de l'amino-diacide.

7. Procédé selon l'une quelconque des revendications précédentes, **caractérisé en ce que** l'acide alcanesulfonique est l'acide méthanesulfonique.

8. Procédé selon l'une quelconque des revendications précédentes, **caractérisé en ce que** la quantité d'acide alcanesulfonique utilisée est choisie dans la gamme allant de 1,01 à 2 moles par mole de l'amino-diacide.

9. Procédé selon l'une quelconque des revendications précédentes, **caractérisé en ce que** le solvant de la réaction est choisi parmi les hydrocarbures aliphatiques ou aromatiques, halogénés ou non.

10. Procédé selon l'une quelconque des revendications précédentes, **caractérisé en ce qu'**on obtient l'ester ω-benzylique de l'amino-diacide sous forme libre en mettant l'alcanesulfonate de l'ester ω-benzylique de l'amino-diacide obtenu en contact avec une base organique ou minérale.

11. Procédé selon la revendication 10, **caractérisé en ce qu'**on utilise la base en quantité suffisante pour atteindre le point isoélectrique de l'ester à obtenir.

12. Procédé selon la revendication 10 ou 11, **caractérisé en ce que** la base est une solution aqueuse d'ammoniaque.

**13.** Procédé selon l'une des revendications précédentes, **caractérisé en ce que** l'on fait cristalliser l'alcanesulfonate de l'ester ω-benzylique de l'amino-diacide avant de le transformer en ester ω-benzylique de l'amino-diacide libre.

**14.** Procédé selon l'une quelconque des revendications précédentes, **caractérisé en ce qu'**on distille l'azéotrope solvant-eau à une température inférieure à 80°C.

**15.** Procédé selon l'une quelconque des revendications précédentes, **caractérisé en ce qu'**on isole l'alcanesulfonate de l'ester ω-benzylique de l'amino-diacide avant de le mettre en contact avec la base.

**16.** Procédé selon l'une quelconque des revendications 1 à 14, **caractérisé en ce qu'**on isole pas du milieu l'alcanesulfonate de l'ester ω-benzylique de l'amino-diacide avant de libérer cet ester.

**17.** Procédé selon l'une quelconque des revendications précédentes, **caractérisé en ce qu'**on dissout avec de l'eau l'alcanesulfonate de l'ester ω-benzylique à transformer en ester libre.

**18.** Procédé selon l'une quelconque des revendications précédentes, **caractérisé en ce qu'**on ajoute un solvant du dérivé de l'alcool benzylique, dans le milieu contenant l'ester à libérer.

**19.** Procédé selon l'une quelconque des revendications précédentes, **caractérisé en ce que** après avoir atteint le pH du point isoélectrique, on chauffe le milieu.

**20.** Alcanesulfonate d'ester ω-benzylique d'amino-diacide carboxylique.

**21.** Alcanesulfonate selon la revendication 20, **caractérisé en ce qu'**il est représenté par la formule (II) suivante :

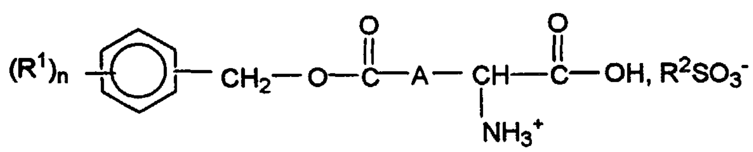

dans laquelle le ou les substituants $R^1$, identiques ou différents, représentent un atome d'hydrogène, un groupe alkyle en $C_1$ à $C_4$, un groupe alcoxy en $C_1$ à $C_4$, ou un atome d'halogène, et n est égal à 1, 2 ou 3, A est la partie de la molécule d'un α-amino-acide carboxylique attachée au carbone en α et au groupe carboxyle en ω et $R^2$ représente le reste alcane de l'acide alcanesulfonique.

**22.** Alcanesulfonate selon la revendication précédente, **caractérisé en ce que** c'est le méthanesulfonate du γ-glutamate de benzyle ou le méthanesulfonate du β-aspartate de benzyle.

Office européen des brevets

# RAPPORT DE RECHERCHE EUROPEENNE

Numéro de la demande
EP 03 29 3080

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| D,A | CLAYTON, D. W. ET AL: "Peptides. Part V. Condensation of the gamma-Carboxyl Group of alpha-Glutamyl Peptides with the Peptide Chain" JOURNAL CHEMICAL SOCIETY, 1956, pages 371-380, XP002254230 * page 374, alinéa 3 * ----- | 1,20 | C07C227/18 C07C229/24 |
| A | STEIN K. A.; TOOGOOD, P. L.: "Enyzme-Catalyzed Regioselective Hydrolysis of Aspartate Diesters" JOURNAL OF ORGANIC CHEMISTRY, vol. 60, no. 24, 1995, pages 8110-8112, XP002254231 * page 8111, alinéa 7 - page 8112, alinéa 1 * ----- | 1,20 | |

DOMAINES TECHNIQUES RECHERCHES (Int.Cl.7)

C07C

Le présent rapport a été établi pour toutes les revendications

| Lieu de la recherche | Date d'achèvement de la recherche | Examinateur |
|---|---|---|
| La Haye | 28 mai 2004 | Delanghe, P |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)